# EUROPÄISCHE PATENTANMELDUNG

(11) **EP 4 361 918 A1**
(43) Veröffentlichungstag der Anmeldung: **01.05.2024**
(21) Anmeldenummer: 23199061.5
(22) Anmeldetag: 22.09.2023
(51) Int. Cl.: G06Q 10/063, G06Q 10/101

(54) **VERFAHREN ZUR ERSTELLUNG EINER TABELLE FÜR DIE VERARBEITUNG VON ATTRIBUTEN VON KOMPONENTEN AUS EINEM LABOR- UND/ODER PROZESSBEREICH**

(30) Priorität: 26.10.2022 DE 102022128367
(71) Anmelder: Analytik Jena GmbH+Co. KG, 07745 Jena (DE)
(72) Erfinder: Taubert, Nadine, 07743 Jena (DE); Oehme, Heiko, 07749 Jena (DE); Schreiber, Andreas, 73525 Schwäbisch Gmünd (DE); Grüneberg, Julian, 73527 Schwäbisch Gmünd (DE); Van, Vinh, 07747 Jena (DE)
(74) Vertreter: Koslowski, Christine Adelheid

(57) **Zusammenfassung**

Die Erfindung bezieht sich auf ein Verfahren zur Erstellung einer Tabelle (T) für die Verarbeitung von Attributen (An) von Komponenten (K) aus einem Labor- und/oder Prozessbereich, wobei die Tabelle (T) aus einer Mehrzahl von Zellen besteht, welche eine Matrix aus Spalten und Zeilen bilden,
wobei jede Zeile jeweils einer Komponente (K) zugeordnet ist und Informationen betreffend die jeweilige Komponente (K) enthält,
wobei jede Spalte einem Attribut (An) zugeordnet ist und Informationen betreffend das Attribut (An) enthält,
wobei einer ersten Spalte ein erstes Attribut (A1) zugeordnet ist, wobei das erste Attribut (A1) eine Menge an Werten (Wn) umfasst, wobei die Menge alle Werte (Wn) des ersten Attributs (A1) für alle Komponenten (K) beinhaltet,
wobei das Verfahren mindestens die folgenden Schritte aufweist:
- Einfügen eines ersten Werts (W1) aus der Menge des ersten Attributs (A1) in eine erste Zelle einer ersten Zeile und der ersten Spalte, wobei der erste Wert die der ersten Zeile zugeordneten Komponente (K1) betrifft, und
- Einfügen mindestens eines zweiten Werts (W2) aus der Menge des ersten Attributs (A1) in die erste Zelle, wobei der zweite Wert (W2) die der ersten Zeile zugeordneten Komponente (K1) betrifft.

## Beschreibung

Die Erfindung betrifft ein Verfahren zur Erstellung einer Tabelle für die Verarbeitung von Attributen von Komponenten aus einem Labor- und/oder Prozessbereich, wobei die Tabelle aus einer Mehrzahl von Zellen besteht, welche eine Matrix aus Spalten und Zeilen bilden. Die Erfindung betrifft ferner ein Computerprogrammprodukt zur Erstellung einer Tabelle für die Verarbeitung von Attributen von Komponenten aus einem Labor- und/oder Prozessbereich, sowie ein computerlesbares Medium. Weiterhin betrifft die Erfindung ein Verfahren zur Verarbeitung von Attributen von Komponenten aus einem Labor- und/oder Prozessbereich mittels einer Tabelle, sowie eine Verwendung einer Tabelle zur Verarbeitung der Attribute der Komponenten und/oder Planung von Prozessen im Labor- und/oder Prozessbereich und eine Verwendung einer Tabelle zur Überwachung der Attribute der Komponenten während der automatisierten Ausführung eines Arbeitsablaufes.

Der Prozessbereich umfasst dabei Prozessanlagen und Messstellen, welche in der Regel mittels Feldgeräten überwacht werden. Feldgeräte dienen beispielsweise zur Bestimmung und/oder Überwachung des Drucks, des pH-Werts, der Leitfähigkeit und/oder des Durchflusses. Eine Probe kann aus der Prozessanlage oder Messstelle entnommen oder vor Ort, d.h. im Prozess, untersucht werden. Der Laborbereich umfasst Laborgeräte, denen Proben zur Untersuchung bereitgestellt werden. Laborgeräte sind insbesondere Analysegeräte, welche die Proben, u.a. hinsichtlich ihrer Zusammensetzung und/oder Konzentration, untersuchen.

Im Labor- und Prozessbereich werden täglich ein hoher Durchsatz von Messungen an Proben durchgeführt. Dies erfordert nicht nur dafür entsprechend ausgelegte Mess- und Laborgeräte, sondern auch Mittel zum Darstellen und Auswerten der Messergebnisse. Aufgrund der Vielzahl an zu untersuchenden Proben und der hohen Zahl an verfügbaren Messmethoden kann die Darstellung der Messergebnisse schnell unübersichtlich werden.

Beispielsweise kann eine einzelne Probe mit einer ganzen Reihe an analytischen Messmethoden untersucht werden, deren Messergebnisse in Form einer Tabelle dargestellt werden. Dabei ist es in der Regel zu unübersichtlich, für jede Messmethode eine eigene Spalte bereitzustellen. Bisherige Tabellen zur Darstellung der Messergebnisse weisen daher Untertabellen auf, welche durch Auswählen eines Tabellenelements angezeigt und ausgeblendet werden können und in welchen die Messergebnisse angezeigt werden.

Die Aufgabe der vorliegenden Erfindung ist es daher, ein Verfahren zur Erstellung einer Tabelle für die Verarbeitung von Attributen von Komponenten aus einem Labor- und/oder Prozessbereich bereitzustellen, welches eine platzsparende und übersichtliche Tabelle generiert.

Die Aufgabe wird erfindungsgemäß gelöst durch ein Verfahren zur Erstellung einer Tabelle für die Verarbeitung von Attributen von Komponenten aus einem Labor- und/oder Prozessbereich nach Anspruch 1, ein Computerprogrammprodukt zur Erstellung einer Tabelle für die Verarbeitung von Attributen von Komponenten aus einem Labor- und/oder Prozessbereich nach Anspruch 10, ein computerlesbares Medium nach Anspruch 11, ein Verfahren zur Verarbeitung von Attributen von Komponenten aus einem Labor- und/oder Prozessbereich mittels einer Tabelle nach Anspruch 12, eine Verwendung einer Tabelle zur Verarbeitung der Attribute der Komponenten und/oder Planung von Prozessen im Labor- und/oder Prozessbereich nach Anspruch 14 und eine Verwendung einer Tabelle zur Überwachung der Attribute der Komponenten während der automatisierten Ausführung eines Arbeitsablaufes nach Anspruch 15.

Die Aufgabe wird erfindungsgemäß gelöst durch ein Verfahren zur Erstellung einer Tabelle für die Verarbeitung von Attributen von Komponenten aus einem Labor- und/oder Prozessbereich, wobei die Tabelle aus einer Mehrzahl von Zellen besteht, welche eine Matrix aus Spalten und Zeilen bilden,
wobei jede Zeile jeweils einer Komponente zugeordnet ist und Informationen betreffend die jeweilige Komponente enthält,
wobei jede Spalte einem Attribut zugeordnet ist und Informationen betreffend das Attribut enthält,
wobei einer ersten Spalte ein erstes Attribut zugeordnet ist, wobei das erste Attribut eine Menge an Werten umfasst, wobei die Menge alle Werte des ersten Attributs für alle Komponenten beinhaltet,
wobei das Verfahren mindestens die folgenden Schritte aufweist:
   - Einfügen eines ersten Werts aus der Menge des ersten Attributs in eine erste Zelle einer ersten Zeile und der ersten Spalte, wobei der erste Wert die der ersten Zeile zugeordneten Komponente betrifft, und
   - Einfügen mindestens eines zweiten Werts aus der Menge des ersten Attributs in die erste Zelle, wobei der zweite Wert die der ersten Zeile zugeordneten Komponente betrifft.

Indem dem ersten Attribut eine Menge an Werten zugeordnet wird, welche alle Werte des ersten Attributs für alle Komponenten umfasst, und in den Zellen jeweils nur die jeweilige Komponente betreffenden Werte eingefügt werden, werden redundante Spalten eingespart. Wäre dem ersten Attribut keine Menge an Werten zugeordnet, so müssten die Werte jeweils in einer Spalte dargestellt werden, was zu einer großen Anzahl an Spalten führen würde. Zudem könnten jeweils eine oder mehrere Zellen der Spalten leerbleiben, da nicht jeder Komponente ein Wert der Spalte zuordenbar wäre. Durch das Zusammenfassen der Werte aller Komponenten innerhalb des ersten Attributs wird lediglich die erste Spalte benötigt, so dass erheblich Platz innerhalb der Tabelle eingespart wird. Die Werte der Attribute sind den Komponenten zuordenbar. Ein Attribut ist ein Oberbegriff für den Komponenten zuordenbare Informationen.

Bevorzugt werden als Komponenten Proben, Laborgeräte und/oder Messgeräte verwendet. Die Proben können im Labor und/oder im Prozess untersucht werden. Als Labor- und/oder Messgeräte werden alle Arten an Geräten bezeichnet, welche eine Probe mittels einer geeigneten Methode untersuchen bzw. analysieren. Als Messgeräte werden zudem auch Feldgeräte bezeichnet, wie sie beispielsweise im Prozessbereich eingesetzt werden.

In einer Ausgestaltung werden der mindestens erste und zweite Wert in Form von Kacheln dargestellt. Als Kacheln werden Objekte mit einer vorgebbaren Form bezeichnet, in welche der mindestens erste und zweite Wert eingetragen werden. Die Kacheln können eine rechteckige, eine ovale oder auch andere Formen aufweisen.

In einer Weiterbildung werden die Kacheln durch unterschiedliche Farben dargestellt, hinterlegt oder umrahmt. Die Kacheln können gänzlich farbig gestaltet sein oder einen farbigen Rahmen aufweisen.

In einer weiteren Ausgestaltung wird den Kacheln eine Kopf- und/oder Fußzeile zugeordnet, welche oberhalb oder unterhalb des jeweiligen Wertes angeordnet sind und in welche eine Zusatzinformation betreffend den jeweiligen Wert eingefügt wird. Durch die Nutzung der Kopf- und/oder Fußzeile für eine Zusatzinformation wird weniger Platz in der Tabelle benötigt, als wenn für die Zusatzinformation eine zusätzliche Zelle verwendet würde. Diese Zusatzinformation wird innerhalb der jeweiligen Kachel (z.B. in dem farbig hinterlegten Bereich der Kachel) angezeigt.

Eine weitere Ausgestaltung sieht vor, dass das erste Attribut eine Menge von Parametern umfasst, wobei in der Kopf- und/oder Fußzeile der jeweilige Parameter und als Wert ein Wert, z.B. ein Messwert, des jeweiligen Parameters angeben wird. Beispielsweise sind die Parameter eine Temperatur oder ein Gas, bei/in welchem eine Probe untersucht wurde. Die Parameter können sich jedoch auch auf Labor- und/oder Messgeräte beziehen, welche mittels der Parameter eingestellt wurden.

Eine alternative Ausgestaltung sieht vor, dass das erste Attribut eine Menge von Labor- und/oder Prozessmethoden umfasst, wobei die Kopf- und/oder Fußzeile die jeweilige Labor- und/oder Prozessmethode und als Wert ein Messergebnis der jeweiligen Labor- und/oder Prozessmethode angegeben wird. Beispielsweise wird in der Kopf- und/oder Fußzeile als Labor- und/oder Prozessmethode eine Stickstoffgehalts- oder eine Chlorgehaltsmessung einer Probe angegeben, und als Wert der jeweilig durch die Messung bestimmte Gehalt an Stickstoff oder Chlor in der Probe.

In einer Weiterbildung werden als erstes Attribut Labor- und/oder Prozessmethoden, Parameter, Gerätestatus, Prozessaufträge, Workflows, Messergebnisse, Kalibrierungsinformationen, statistische Auswertungen, Qualitätskontrollinformation, Belegungsinformationen von Geräten, und/oder Wartungsinformation verwendet. Beispielsweise umfasst das erste Attribut eine Menge von allen verfügbaren Labor- und/oder Prozessmethoden oder Messergebnissen. Hinsichtlich der Wartungsinformation kann das erste Attribut beispielsweise jeweils mindestens eine Wartungsinformation für jede Komponente umfassen. Hinsichtlich der Belegungsinformation von Geräten kann das erste Attribut eine definierte Anzahl an Positionen von Proben und/oder die Proben enthaltenen Behältnissen umfassen. Hinsichtlich des Gerätestatus kann das erste Attribut mindestens eine Information über den Gerätestatus für jedes Labor- und/oder Prozessgerät umfassen. Hinsichtlich der Qualitätskontrollinformation kann das erste Attribut mindestens eine Qualitätskontrollinformation für jede Labor- und/oder Prozessmethode umfassen. Hinsichtlich der statistischen Auswertungen kann das erste Attribut für jede Labor- und/oder Prozessmethode mindestens eine statistische Information umfassen. Hinsichtlich der Kalibrierungsinformation kann das erste Attribut für jeden Messwert mindestens eine Kalibrierungsinformation umfassen. Hinsichtlich der Parameter kann das erste Attribut für jede Labor- und/oder Prozessmethode mindestens einen Parameter umfassen. Hinsichtlich der Prozessaufträge und Workflows kann das erste Attribut für jede Labor- und/oder Prozessmethode eine Probe oder mehrere Proben umfassen.

In einer anderen Ausgestaltung wird zumindest einer der mindestens ersten und zweiten Werte ausgeblendet und stattdessen ein Symbol angezeigt, wobei durch Auswählen des Symbols der vorher ausgeblendete Wert angezeigt wird.

Die objektive Aufgabe wird weiterhin erfindungsgemäß gelöst durch ein Computerprogrammprodukt zur Erstellung einer Tabelle für die Verarbeitung von Attributen von Komponenten aus einem Labor- und/oder Prozessbereich, mit computerlesbaren Programmcodeelementen, die bei der Ausführung des Computerprogramms durch einen Computer diesen veranlassen, das Verfahren nach mindestens einem der vorherigen Ansprüche auszuführen. Da mittels des ersten Attributs die erstellte Tabelle weniger Platz beansprucht als alternative Lösungen, beansprucht das erfindungsgemäße Computerprogrammprodukt weniger Rechenleistung und Speicherkapazität.

Ferner wird die Aufgabe erfindungsgemäß gelöst durch ein computerlesbares Medium, auf dem zumindest das Computerprogramm nach Anspruch 10 gespeichert ist.

Die Aufgabe wird zudem erfindungsgemäß gelöst durch ein Verfahren zur Verarbeitung von Attributen von Komponenten aus einem Labor- und/oder Prozessbereich mittels einer Tabelle, wobei die Tabelle aus einer Mehrzahl von Zellen besteht, welche eine Matrix aus Spalten und Zeilen bilden,
wobei jede Zeile jeweils einer Komponente zugeordnet ist und Informationen betreffend die jeweilige Komponente enthält,
wobei jede Spalte einem Attribut zugeordnet ist und Informationen betreffend das Attribut enthält,
wobei einer ersten Spalte ein erstes Attribut zugeordnet ist, wobei das erste Attribut eine Menge an Werten umfasst, wobei die Menge alle Werte des ersten Attributs für alle Komponenten beinhaltet,
wobei das Verfahren zumindest folgende Schritte aufweist:
   - Einfügen von Werten in die Tabelle, wobei die Werte in den Komponenten und/oder in einer übergeordneten Einheit bereitgestellt werden, wobei nur diejenigen Werte aus der Menge an Werten in die Zeile der jeweiligen Komponente eingetragen werden, welche die jeweilige Komponente betreffen,
   - Evaluieren der in die Tabelle eingefügten Werte für mindestens eine der Komponenten anhand der Tabelle, und
   - Basierend auf einem Ergebnis der Evaluierung, Ableiten mindestens einer Maßnahme für die mindestens eine Komponente.

Die Komponenten können die in die Tabelle einzufügenden Werte entweder selbst bereitstellen und/oder die Werte werden in einer übergeordneten Einheit bereitgestellt, wie beispielsweise einer Prozesskontrolleinheit oder einer Cloud, mit welcher die Komponenten verbunden sind. Indem das erste Attribut in der ersten Spalte alle Werte des ersten Attributs für alle Komponenten beinhaltet und nur diejenigen Werte in die Tabelle eingefügt sind, welche die jeweilige Komponente betreffen, wird erreicht, dass die Tabelle möglichst platzsparend und übersichtlich gehalten wird. Mittels der platzsparenden Tabelle können die den Komponenten zugeordneten Werte somit auf einfache Weise evaluiert werden. Als Maßnahme kann mittels der Evaluierung beispielsweise abgeleitet werden, mit welcher Labor- und/oder Prozessmethode eine Probe in einem nächsten Schritt untersucht werden soll.

In einer Weiterbildung des erfindungsgemäßen Verfahrens werden die Werte in der Tabelle kontinuierlich oder nach vorgebbaren Zeitabständen aktualisiert.

Weiterhin wird die Aufgabe erfindungsgemäß gelöst durch eine Verwendung einer Tabelle zur Verarbeitung von Attributen von Komponenten im Labor- und/oder Prozessbereich und/oder zur Planung von Prozessen im Labor- und/oder Prozessbereich, wobei die Tabelle aus einer Mehrzahl von Zellen besteht, welche eine Matrix aus Spalten und Zeilen bilden,
wobei jede Zeile jeweils einer Komponente zugeordnet ist und Informationen betreffend die jeweilige Komponente enthält,
wobei jede Spalte einem Attribut zugeordnet ist und Informationen betreffend das Attribut enthält,
wobei einer ersten Spalte ein erstes Attribut zugeordnet ist, wobei das erste Attribut eine Menge an Werten umfasst, wobei die Menge alle Werte des ersten Attributs für alle Komponenten beinhaltet,
wobei nur diejenigen Werte aus der Menge an Werten in die Zeile der jeweiligen Komponente eingetragen sind, welche die jeweilige Komponente betreffen.

Mittels der platzsparenden Tabelle können die Attribute der Komponenten auf einfache Weise durch ein Computerprogramm oder einen Benutzer verarbeitet werden. Ebenso erlaubt die Tabelle eine einfache Planung von Prozessen im Labor- und/oder Prozessbereich, insbesondere hinsichtlich Wartungsarbeiten und Workflows.

Ferner wird die Aufgabe erfindungsgemäß gelöst durch eine Verwendung einer Tabelle zur Überwachung der Attribute der Komponenten während der automatisierten Ausführung eines Arbeitsablaufes anhand von in die Tabelle eingefügten Werten, wobei die Tabelle aus einer Mehrzahl von Zellen besteht, welche eine Matrix aus Spalten und Zeilen bilden,
wobei jede Zeile jeweils einer Komponente zugeordnet ist und Informationen betreffend die jeweilige Komponente enthält,
wobei jede Spalte einem Attribut zugeordnet ist und Informationen betreffend das Attribut enthält,
wobei einer ersten Spalte ein erstes Attribut zugeordnet ist, wobei das erste Attribut eine Menge an Werten umfasst, wobei die Menge alle Werte des ersten Attributs für alle Komponenten beinhaltet,
wobei nur diejenigen Werte aus der Menge an Werten in die Zeile der jeweiligen Komponente eingetragen sind, welche die jeweilige Komponente betreffen.

Die platzsparende Tabelle lässt sich auch vorteilhaft zum Überwachen von Attributen während der automatisierten Ausführung eines Arbeitsablaufes einsetzen. So kann anhand der in die Tabelle eingefügten Werte erkannt werden, ob eine eingestellte Messung bereits durchgeführt wurde und die Messergebnisse in die Tabelle eingetragen sind oder ob die gewünschten Parameter bei der jeweiligen Messung verwendet wurden. Beispielsweise können in der Kopf- oder Fußzeile auch Fortschritts- oder Statusinformationen anzeigt werden. Des Weiteren können durch die Wahl geeigneter Farben für die Kacheln auch Informationen zur Gültigkeit der Attribute dargestellt werden. So lassen sich fragwürdige oder ungültige Messergebnisse mit einer Signalfarbe (z.B. Rot) hinterlegen.

Im Weiteren wird die vorliegende Erfindung anhand der nachfolgenden Figuren Fig. 1-3 näher erläutert werden. Sie zeigen:
Fig. 1: eine schematische Darstellung einer Tabelle, welche in Verbindung mit der vorliegenden Erfindung genutzt wird.
Fig. 2: eine weitere schematische Darstellung einer Tabelle, welche in Verbindung mit der vorliegenden Erfindung genutzt wird.
Fig: 3: ein Beispiel für eine Tabelle, welche in Verbindung mit der vorliegenden Erfindung genutzt wird.

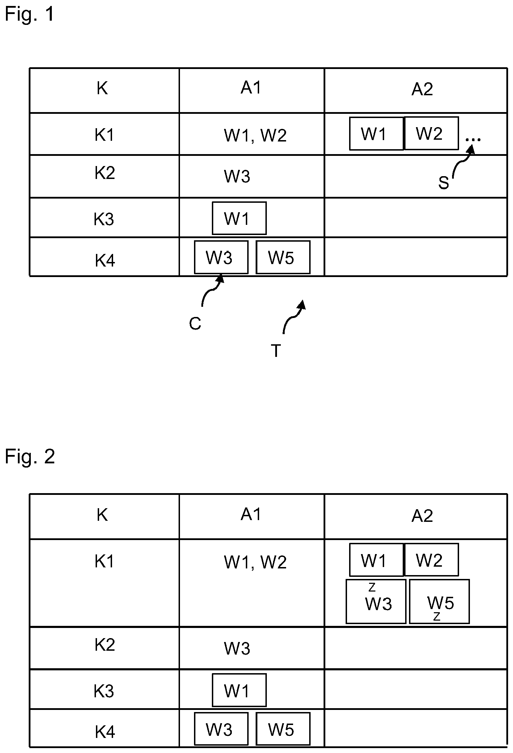

Fig. 1 zeigt ein Beispiel für eine Tabelle, welche in Verbindung mit den beschriebenen erfindungsgemäßen Verfahren und Verwendungen genutzt wird. Die Tabelle besteht aus einer Mehrzahl an Zellen, welche eine Matrix aus Spalten und Zeilen bilden. Jede Zeile ist einer Komponente K1, K2, ... zugeordnet und enthält Information betreffend die jeweilige Komponente K1, K2.... Die Spalten sind jeweils einem Attribut An zugeordnet und enthalten Informationen betreffend das Attribut An. Der ersten, in Fig. 1 mittleren Spalte ist ein erstes Attribut A1 zugeordnet, einer zweiten, in Fig. 1 rechts angeordneten Spalte ein zweites Attribut A2. Das erste Attribut A1 umfasst eine Menge an Werten Wn, welche Menge alle Werte des ersten Attributs A1 für alle Komponenten K beinhaltet. In analoger Weise umfasst das zweite Attribut A2 hier beispielhaft eine weitere Menge an Werten Wn.

Entsprechend dem erfindungsgemäßen Verfahren zur Erstellung einer Tabelle für die Verarbeitung von Attributen von Komponenten aus einem Labor- und/oder Prozessbereich ist in die erste Zelle der ersten Spalte und der ersten Zeile ein erster Wert W1 eingefügt, welcher die der ersten Zeile zugeordneten Komponente K1 betrifft. Zusätzlich ist ein zweiter Wert W2 in dieselbe Zelle eingefügt, welcher ebenfalls die Komponente K1 betrifft.

Die erste, dem ersten Attribut A1 zugehörige Spalte zeigt noch weitere Werte W3, W5, welche anderen Komponenten K2, K4 zugeordnet sind. Die dem ersten Attribut A1 zugeordnete Menge an Werten Wn kann dabei noch weitere Werte Wn umfassen. Indem alle Werte Wn dem ersten Attribut A1 zugeordnet werden, genügt es, in die erste Spalte lediglich diejenigen Werte in die Zeile der jeweiligen Komponente einzufügen, die der jeweiligen Komponente zugeordnet sind. Wären die Werte Wn nicht dem ersten Attribut A1 zugeordnet, so müsste beispielsweise für jeden Wert Wn eine eigene Spalte generiert werden, so dass die Tabelle sehr groß und unübersichtlich würde. Alternativ müsste auf Untertabellen zurückgegriffen werden, welche innerhalb oder neben der Tabelle nach Wunsch angezeigt werden können. Mithilfe des erfindungsgemäßen Verfahrens wird hingegen eine kompakte und übersichtliche Tabelle erstellt.

In Fig. 1 sind zudem manche der in die Tabelle einfügten Werte Wn in Form von Kacheln C dargestellt. In diesem Beispiel sind die Kacheln rechteckig und weisen einen dunklen Umriss auf. Selbstverständlich sind auch andere Formen und Farben denkbar. In der dem zweiten Attribut A2 zugeordneten Spalte ist zudem ein optionales Symbol S in Form von drei Punkten gezeigt. Durch Auswählen des Symbols S werden vormals ausgeblendete Werte Wn angezeigt, wie in Fig. 2 gezeigt. Fig. 2 ist mit Fig. 1 identisch und zeigt die Darstellung der Tabelle nach Auswählen des Symbols S. Durch Auswählen der entsprechenden Zelle und/oder der in der Zelle vorhandenen Werte Wn kann wieder in die Darstellung der Fig. 1 gewechselt werden.

Die vormals ausgeblendeten Werte W3, W5 in der dem zweiten Attribut S2 zugehörigen Spalte in Fig. 2 weisen zudem eine Kopf- und/oder Fußzeile auf. Diese sind jeweils oberhalb oder unterhalb des Wertes angeordnet und geben eine Zusatzinformation Z wieder, welchen den jeweiligen Wert betrifft.

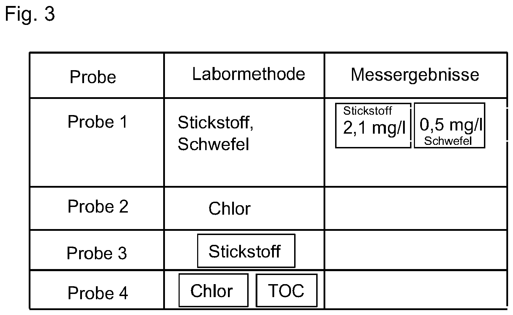

Fig. 3 zeigt ein konkretes Ausführungsbeispiel einer Tabelle T, in welcher mehrere Proben 1-4 als Attribute An auf die Probe angewandte Labormethoden und deren Messergebnisse zugeordnet sind. In der Tabelle ist dabei eingefügt, welche Labormethode auf die jeweilige Probe angewendet wurde und welches das dabei bestimmte Messergebnis ist, insofern eine Auswertung schon stattgefunden hat. So wurde beispielsweise für die Probe 1 bereits der Stickstoff- und der Schwefelgehalt bestimmt und für die Probe 2 der Chlorgehalt. In der Spalte mit den Messergebnissen ist das mittels der in der Kopf- und oder Fußzeile angegebenen Methode bestimmte Messergebnis angegeben.

Hinsichtlich des erfindungsgemäßen Verfahrens zur Verarbeitung von Attributen An von Komponenten K aus einem Labor- und/oder Prozessbereich mittels einer Tabelle T kann ein Anwender nun die Attribute An, also die Labormethoden und die Messergebnisse, und insbesondere deren Werte evaluieren und eine Maßnahme für eine der Proben ableiten. Beispielsweise kann der Anwender bei der Evaluierung feststellen, dass der Chlorgehalt für Probe 2 noch nicht ausgewertet wurde und als Maßnahme ableiten die Auswertung nun durchzuführen. Alternativ kann der Anwender die Attribute für Probe 3 evaluieren und als Maßnahme die noch ausstehende Durchführung der Bestimmung des TOC-Gehalts (total organic carbon, Gesamtkohlenstoffgehalt) bestimmt werden muss.

Erfindungsgemäß lässt sich die Tabelle dazu verwenden, um Attribute An von Komponenten K im Labor und/oder Prozessbereich und/oder Prozesse im Labor- und/oder Prozessbereich zu planen und zu überwachen. Dabei kann die Tabelle zur Überwachung der Attribute während jedes Arbeitsschritts im Workflow betrachtet werden und dieser ggf. angepasst werden. Wird beispielsweise erkannt, dass eine Reihe an Proben ungültige Ergebnisse liefern, so kann die Abfolge der zu messenden Proben derart angepasst werden, dass zunächst eine andere Messreihe untersucht wird, bei der ggf. vorteilhaftere Messergebnisse erhalten werden können. Die Anpassung der Messreihenfolge kann beispielsweise durch die Verschiebung der Kacheln in einer Zelle durchgeführt werden.

### Bezugszeichenliste

- T: Tabelle
- K: Komponente
- An: n-tes Attribut
- Wn: n-ter Wert
- C: Kachel
- S: Symbol
- Z: Zusatzinformation

## Patentansprüche

1. Verfahren zur Erstellung einer Tabelle (T) für die Verarbeitung von Attributen (An) von Komponenten (K) aus einem Labor- und/oder Prozessbereich, wobei die Tabelle (T) aus einer Mehrzahl von Zellen besteht, welche eine Matrix aus Spalten und Zeilen bilden,
wobei jede Zeile jeweils einer Komponente (K) zugeordnet ist und Informationen betreffend die jeweilige Komponente (K) enthält,
wobei jede Spalte einem Attribut (An) zugeordnet ist und Informationen betreffend das Attribut (An) enthält,
wobei einer ersten Spalte ein erstes Attribut (A1) zugeordnet ist, wobei das erste Attribut (A1) eine Menge an Werten (Wn) umfasst, wobei die Menge alle Werte (Wn) des ersten Attributs (A1) für alle Komponenten (K) beinhaltet,
wobei das Verfahren mindestens die folgenden Schritte aufweist:
- Einfügen eines ersten Werts (W1) aus der Menge des ersten Attributs (A1) in eine erste Zelle einer ersten Zeile und der ersten Spalte, wobei der erste Wert die der ersten Zeile zugeordneten Komponente (K1) betrifft, und
- Einfügen mindestens eines zweiten Werts (W2) aus der Menge des ersten Attributs (A1) in die erste Zelle, wobei der zweite Wert (W2) die der ersten Zeile zugeordneten Komponente (K1) betrifft.

2. Verfahren nach Anspruch 1,
wobei als Komponenten (K) Proben, Laborgeräte und/oder Messgeräte verwendet werden.

3. Verfahren nach mindestens einem der Ansprüche 1-2,
wobei der mindestens erste Wert (W1) und der mindestens zweite Wert (W2) in Form von Kacheln (C) dargestellt werden.

4. Verfahren nach Anspruch 3,
wobei die Kacheln (C) durch unterschiedliche Farben dargestellt, hinterlegt oder umrahmt werden.

5. Verfahren nach mindestens einem der Ansprüche 3-4,
wobei den Kacheln (C) eine Kopf- und/oder Fußzeile zugeordnet wird, welche oberhalb oder unterhalb des jeweiligen Wertes (Wn) angeordnet sind und in welche eine Zusatzinformation (Z) betreffend den jeweiligen Wert (Wn) eingefügt wird.

6. Verfahren nach Anspruch 5,
wobei das erste Attribut (A1) eine Menge von Parametern umfasst, wobei in der Kopf- und/oder Fußzeile der jeweilige Parameter und als Wert (Wn) ein Wert des jeweiligen Parameters angeben wird.

7. Verfahren nach Anspruch 5,
wobei das erste Attribut (A1) eine Menge von Labor- und/oder Prozessmethoden umfasst, wobei die Kopf- und/oder Fußzeile die jeweilige Labor- und/oder Prozessmethode und als Wert (Wn) ein Ergebnis der jeweiligen Labor- und/oder Prozessmethode angegeben wird.

8. Verfahren nach mindestens einem der Ansprüche 1-7,
wobei als erstes Attribut (A1) Labor- und/oder Prozessmethoden, Parameter, Gerätestatus, Prozessaufträge, Workflows, Messergebnisse, Kalibrierungsinformationen, statistische Auswertungen, Qualitätskontrollinformationen, Belegungsinformationen von Geräten, und/oder Wartungsinformation verwendet werden.

9. Verfahren nach mindestens einem der Ansprüche 1-8,
wobei zumindest einer der mindestens ersten und zweiten Werte (W1, W2) ausgeblendet und stattdessen ein Symbol (S) angezeigt wird, wobei durch Auswählen des Symbols (S) der vorher ausgeblendete Wert (W1, W2) angezeigt wird.

10. Computerprogrammprodukt zur Erstellung einer Tabelle (T) für die Verarbeitung von Attributen (An) von Komponenten (K) aus einem Labor- und/oder Prozessbereich, mit computerlesbaren Programmcodeelementen, die bei der Ausführung des Computerprogramms durch einen Computer diesen veranlassen, das Verfahren nach mindestens einem der vorherigen Ansprüche auszuführen.

11. Computerlesbares Medium, auf dem zumindest das Computerprogramm nach Anspruch 10 gespeichert ist.

12. Verfahren zur Verarbeitung von Attributen (An) von Komponenten (K) aus einem Labor- und/oder Prozessbereich mittels einer Tabelle (T), wobei die Tabelle (T) aus einer Mehrzahl von Zellen besteht, welche eine Matrix aus Spalten und Zeilen bilden,
wobei jede Zeile jeweils einer Komponente (K) zugeordnet ist und Informationen betreffend die jeweilige Komponente (K) enthält,
wobei jede Spalte einem Attribut (An) zugeordnet ist und Informationen betreffend das Attribut (An) enthält,
wobei einer ersten Spalte ein erstes Attribut (A1) zugeordnet ist, wobei das erste Attribut (A1) eine Menge an Werten (Wn) umfasst, wobei die Menge alle Werte (Wn) des ersten Attributs (A1) für alle Komponenten (K) beinhaltet,
wobei das Verfahren zumindest folgende Schritte aufweist:
- Einfügen von Werten (Wn) in die Tabelle (T), wobei die Werte (Wn) in den Komponenten (K) und/oder in einer übergeordneten Einheit bereitgestellt werden, wobei nur diejenigen Werte (Wn) aus der Menge an Werten (Wn) in die Zeile der jeweiligen Komponente (K) eingetragen werden, welche die jeweilige Komponente (K) betreffen,
- Evaluieren der in die Tabelle (T) eingefügten Werte (Wn) für mindestens eine der Komponenten (K) anhand der Tabelle (T), und
- Basierend auf einem Ergebnis der Evaluierung, Ableiten mindestens einer Maßnahme für die mindestens eine Komponente (K).

13. Verfahren nach Anspruch 14,
wobei die Werte (Wn) in der Tabelle (T) kontinuierlich oder nach vorgebbaren Zeitabständen aktualisiert werden.

14. Verwendung einer Tabelle (T) zur Verarbeitung von Attributen (An) von Komponenten (K) im Labor und/oder Prozessbereich und/oder zur Planung von Prozessen im Labor- und/oder Prozessbereich, wobei die Tabelle (T) aus einer Mehrzahl von Zellen besteht, welche eine Matrix aus Spalten und Zeilen bilden, wobei jede Zeile jeweils einer Komponente (K) zugeordnet ist und Informationen betreffend die jeweilige Komponente (K) enthält,
wobei jede Spalte einem Attribut (An) zugeordnet ist und Informationen betreffend das Attribut (An) enthält,
wobei einer ersten Spalte ein erstes Attribut (A1) zugeordnet ist, wobei das erste Attribut (A1) eine Menge an Werten (Wn) umfasst, wobei die Menge alle Werte (Wn) des ersten Attributs (A1) für alle Komponenten (K) beinhaltet,
wobei nur diejenigen Werte aus der Menge an Werten (Wn) in die Zeile der jeweiligen Komponente (K) eingetragen sind, welche die jeweilige Komponente (K) betreffen.

15. Verwendung einer Tabelle (T) zur Überwachung der Attribute (An) der Komponenten (K) während der automatisierten Ausführung eines Arbeitsablaufes anhand von in die Tabelle (T) eingefügten Werten (Wn), wobei die Tabelle (T) aus einer Mehrzahl von Zellen besteht, welche eine Matrix aus Spalten und Zeilen bilden,
wobei jede Zeile jeweils einer Komponente (K) zugeordnet ist und Informationen betreffend die jeweilige Komponente (K) enthält,
wobei jede Spalte einem Attribut (An) zugeordnet ist und Informationen betreffend das Attribut (An) enthält,
wobei einer ersten Spalte ein erstes Attribut (A1) zugeordnet ist, wobei das erste Attribut (A1) eine Menge an Werten (Wn) umfasst, wobei die Menge alle Werte (Wn) des ersten Attributs (A1) für alle Komponenten (K) beinhaltet,
wobei nur diejenigen Werte aus der Menge an Werten (Wn) in die Zeile der jeweiligen Komponente (K) eingetragen sind, welche die jeweilige Komponente (K) betreffen.
